# EUROPÄISCHE PATENTSCHRIFT

(11) **EP 0 850 214 B1**
(45) Veröffentlichungstag und Bekanntmachung des Hinweises auf die Patenterteilung: **05.04.2000**
(21) Anmeldenummer: 96931783.3
(22) Anmeldetag: 09.09.1996
(51) Int. Cl.: C07C 209/62

(54) **SPALTUNG VON OPTISCH AKTIVEN AMIDEN**
SEPARATION OF OPTICALLY ACTIVE AMIDES
DISSOCIATION D'AMIDES OPTIQUEMENT ACTIFS

(30) Priorität: 15.09.1995 DE 19534208
(43) Veröffentlichungstag der Anmeldung: 01.07.1998
(73) Patentinhaber: BASF AKTIENGESELLSCHAFT, 67056 Ludwigshafen (DE)
(72) Erfinder: DITRICH, Klaus, D-67161 Gönnheim (DE); BALKENHOHL, Friedhelm, D-67117 Limburgerhof (DE); LADNER, Wolfgang, D-67136 Fussgönheim (DE)
(86) Internationale Anmeldenummer: EP9603948
(87) Internationale Veröffentlichungsnummer: WO9710201

(56) Entgegenhaltungen:
- WO-A-95/08636
- DE-A- 3 819 438
- US-A- 3 592 854

## Beschreibung

Die vorliegende Erfindung betrifft ein neues Verfahren zur Spaltung von optisch aktiven Amiden.

Die hydrolytische Spaltung von optisch aktiven Amiden, die im Aminteil des Moleküls ein Chiralitätszentrum besitzen, ist nicht oder nur unter sehr aufwendigen Bedingungen so durchführbar, daß das Chiralitätszentrum erhalten bleibt.

Von Devant und Braun (Chem. Berichte 119, 2191-2207 (1986)) wird beschrieben, daß die Abspaltung von chiralen Aminen aus Acetamiden nicht ohne Zerstörung des Chiralitätszentrums möglich ist (Seite 2194). Weiterhin finden die Autoren, daß zahlreiche Versuche, die Amide alkalisch oder sauer zur Carbonsäure und optisch aktivem Amin zu hydrolysieren, erfolglos geblieben sind, und daß lediglich die Umsetzung mit Distickstofftetroxid nach White (J. Am. Chem. Soc. 77, 6008 (1955)) zum gewünschten Ergebnis führt. Diese Umsetzung mit N₂O₄ ist jedoch aufwendig und daher nicht für technische Verfahren geeignet.

In WO 95/08636 wird ein enzymatisches Verfahren zur Racematspaltung von optisch aktiven Aminen beschrieben, bei dem die Amine enantioselektiv mit einem Ester acyliert werden, dann die Mischung aus acyliertem Amin (Amid) und nicht-umgesetzten Amin getrennt wird und gewünschtenfalls aus dem acylierten Amin (Amid) das optisch aktive Amin durch Amidspaltung freigesetzt wird. Es werden jedoch keine Verfahrensparameter angegeben, bei denen die Amidspaltung durchgeführt werden kann.

Es bestand daher die Aufgabe, gerade im Hinblick auf das in WO 95/08636 beschriebene effektive Verfahren zur Racematspaltung von Aminen ein kostengünstiges und technisch gut durchführbares Verfahren zur Hydrolyse von optisch aktiven Amiden unter Erhalt des Chiralitätszentrums bereitzustellen.

Diese Aufgabe wird gelöst durch ein Verfahren zur Spaltung von optisch aktiven Amiden zu Carbonsäuren und optisch aktiven Aminen unter Erhalt des Chiralitätszentrums, das dadurch gekennzeichnet ist, daß man eine Hydrolyse der Amide in Gegenwart eines Polyols oder Aminoalkohols und eines Alkali- oder Erdalkalihydroxids durchführt.

Das erfindungsgemäße Verfahren eignet sich für praktisch alle Amide, die aus optisch aktiven primären oder sekundären Aminen herstellbar sind. Besonders geeignet ist es für Amide, deren Aminteil aus einem optisch aktiven Arylalkylamin besteht.

Besonders gut verläuft es bei primären Arylalkylaminen, beispielsweise solchen mit folgenden Strukturen: 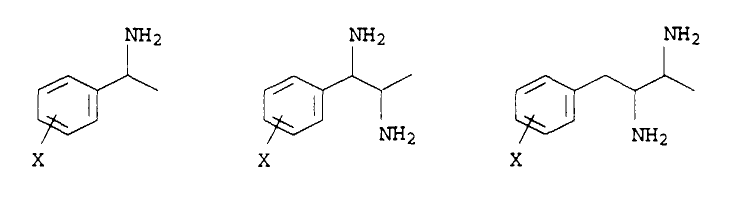 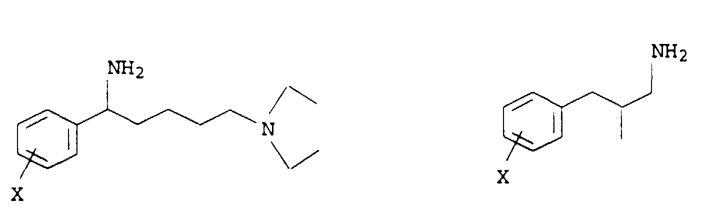 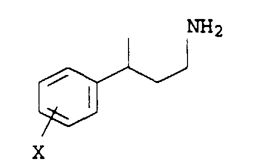 wobei X für alle gängigen Aromatensubstituenten, insbesondere Halogen, Nitro, Cyano, C₁-C₄-Alkyl, C₁-C₄-Alkoxy und C₁-C₄-Alkylthio, steht.

Des weiteren eignet sich das erfindungsgemäße Verfahren zur Spaltung von Amiden, deren Aminteil aus einem Aminoalkohol der allgemeinen Formel besteht 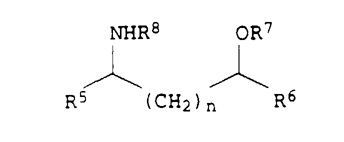 in der die Substituenten folgende Bedeutung haben:
- R⁵, R⁶ =: unabhängig voneinander H, verzweigtes und unverzweigtes C₁-C₁₀-Alkyl, C₁-C₄-Alkoxycarbonyl, Phenyl, Phenyl-C₁-C₄-alkyl, wobei die Phenylgruppen durch Halogen, Nitro, Cyano, C₁-C₄-Alkyl, C₁-C₄-Alkoxy und C₁-C₄-Alkylthio substituiert sein können. Darüber hinaus können R⁵ und R⁶ über eine Kohlenstoffkette, die durch Sauerstoff, Schwefel oder Stickstoff unterbrochen und ihrerseits substituiert sein kann, zu einem Mono-, Bi-oder Tricyclus geschlossen sein
- R⁷ =: H, C₁-C₁₀-Alkyl, C₁-C₄-Alkoxycarbonyl
- R⁸ =: H, C₁-C₁₀-Alkyl
- n =: 0 oder 1.

Sofern die durch OR⁷ bzw. NHR⁸ substituierten Kohlenstoffatome stereogene Zentren sind, bezieht sich das erfindungsgemäße Verfahren sowohl auf die syn- als auch die anti-Isomeren.

Als Beispiele für Aminoalkohole der obigen allgemeinen Struktur seien genannt:
2-Amino-1-butanol; Ephedrin; Pseudoephedrin; Norephedrin; Norpseudoephedrin; tert. Leucinol; Phenylglycidol; 1,2-Diphenylaminoethanol; cis- und trans-2-Aminocyclopentanol; cis- und trans-1-Amino-2-hydroxyindan; cis- und trans-2-Aminocyclohexanol, Statin, 2-Hydroxy-3-amino-phenylpropionsäure.

Als bevorzugte Aminoalkohole sind zu nennen: cis- und trans-1-Amino-2-hydroxyindan.

Als Polyole können in dem erfindungsgemäßen Verfahren Glykole, z.B. Ethylenglykol und seine Monoether-, z.B. Monomethylglykol verwendet werden.

Weitere geeignete Polyole sind Glycerin, 1,2-Propandiol, 1,3-Propandiol, 1,2-Butandiol, 2,3-Butandiol, 2,4-Pentandiol, cis-und trans Cyclohexan-1,2-diol, cis-und trans Cyclohexan-1,4-diol, 2-Methyl-2,3 butandiol, 3-Methyl-2,4-pentandiol, 2,2-Dimethyl-1,3-propandiol,1-Phenyl-1,2-ethandiol, 3-Methoxy-1,2-propandiol, 3-Phenoxy-1,2-propandiol, 3-Buten-1,2-diol, cis- und trans-2-Buten-1,4-diol, Triethanolamin, Triisopropanolamin.

Weiterhin können auch Polyalkylenglykole, bevorzugt Dialkylenglykole und deren Ether insbesondere Diethylenglykol und Diglyme als Polyole eingesetzt werden.

Geeignete Aminoalkohole für die erfindungsgemäße Amidspaltung sind Ethanolamin, Diethanolamin und Triethanolamin.

Die Polyole oder Aminoalkohole sollen in Wasser löslich oder mit Wasser homogen mischbar sein. Es können auch Mischungen verschiedener Polyole oder Aminoalkohole eingesetzt werden.

Bevorzugtes Polyol ist Ethylenglykol.

Die Polyole werden in einer Menge von 10 - 90, bevorzugt von 30 - 80 Gew.-% bezogen auf das gesamte Lösungsmittel bei der Hydrolyse verwendet.

Ein weiterer notwendiger Bestandteil bei der erfindungsgemäßen Spaltung sind Alkali- oder Erdalkalihydroxide, insbesondere Natrium- und Kaliumhydroxid. Diese katalysieren die Hydrolyse, werden jedoch auch durch die entstehende Säure neutralisiert, so daß man sie üblicherweise in einer Menge von 1-10 Äquivalenten bezogen auf Amid einsetzt.

Die Hydroxide können vorteilhaft in Form ihrer wäßrigen Lösungen eingesetzt werden, da bei der erfindungsgemäßen Spaltung ohnehin ein gewisser Wasseranteil benötigt wird. Der Wasseranteil beträgt in der Regel 5 - 90 Gew.-% bezogen auf das gesamte Lösungsmittel. Die erfindungsgemäße Spaltung wird bevorzugt bei Temperaturen oberhalb von 100°C, besonders bevorzugt oberhalb von 150°C durchgeführt.

Eine besonders geeignete Ausführungsform der Erfindung besteht darin, daß man die Spaltung bei einer so hohen Temperatur ausführt, das das erhaltene Reaktionsprodukt (Amin) mit dem Wasserdampf überdestilliert und so gleich aus dem Reaktionsgemisch entfernt wird, während die Säure, die bei den alkalischen Bedingungen dissoziiert vorliegt, in der Vorlage zurückbleibt.

Das erfindungsgemäße Verfahren läßt sich mit gutem Erfolg als Teilschritt (Schritt 3) bei dem in WO 95/08636 beschriebenen Verfahren zur Racematspaltung von primären und sekundären Aminen einsetzen. Dieses Verfahren umfaßt die folgenden Schritte:
1. Umsetzung der racemischen Amine mit einem Ester, dessen Säurekomponente ein Fluor-, Stickstoff-, Sauerstoff-, Phosphor- oder Schwefelatom, gebunden an ein Kohlenstoffatom in alpha, beta oder gamma Position zum Carbonylkohlenstoffatom, trägt, unter spezifischer Katalyse mit einer Hydrolase,
2. Trennung des einen, enantioselektiv acylierten Amins vom nicht umgesetzten anderen Enantiomer des Amins,
3. anschließende Hydrolyse des acylierten Amins.

Die für dieses Verfahren geeigneten Ester sind solche, die in der Säurekomponente des Esters ein elektronenreiches Heteroatom, gebunden an ein Kohlenstoffatom, das sich in alpha, beta oder gamma Position zum Carbonylkohlenstoff befindet, tragen.

Das Heteroatom kann ein Fluor-, Stickstoff-, Sauerstoff-, Phosphor- oder Schwefelatom sein. Als Heteroatom ist Sauerstoff bevorzugt.

Das Heteroatom kann gegebenenfalls mit weiteren Gruppen, z.B. Alkylgruppen, verknüpft sein. Ist das Heteroatom beispielsweise Sauerstoff, so liegt eine Ethergruppe vor.

Die Alkoholkomponente des Esters kann aus verzweigten oder unverzweigten C₁- bis C₁₀-Alkoholen, die auch gegebenenfalls substituiert sein können, bestehen.

Besonders geeignete Alkoholkomponenten sind 2-Propanol, 2-Butanol, 2-Pentanol, 3-Pentanol, 3- Methyl-2-butanol, Cyclopentanol, Cyclohexanol, 2-Methylcyclohexanol, 1-Chlor-2-propanol, 1-Brom-2-Propanol, 4-Methyl-2-pentanol, 2,4-Dimethyl-3-pentanol, Cyclopropylethanol, 1-Phenylethanol, l-Phenoxy-2-propanol, l-Methoxy-2-propanol,cis-und trans 2-Methoxycyclohexanol, l-Dimethylamino-2-propanol, 1-Buten-3-ol, l-Butin-3-ol, 1-Indanol, 2-Indanol, 3-Hydroxytetrahydrofuran, 5-Hydroxy-2-methyl-1,3-di-oxan, 4-Hydroxypiperidin, (+) und (-)-Menthol, (+) und (-)-Isomenthol, Carfenol, Milchsäurenitril, Acetoncyanhydrin, Benzaldehydcyanhydrin, Pantolacton, Milchsäure-t-butylester, Acetonoxim-2-hydroxypropylether.

Weitere geeignete Alkoholkomponenten sind 1,2-Ethandiol, Glycerin, 1,2-Propandiol, 1,3-Propandiol, 1,2-Butandiol, 2,3-Butandiol, 2,4-Pentandiol, cis-und trans Cyclohexan-1,2-diol, cis-und trans Cyclohexan-1,4-diol, 2-Methyl-2,3 butandiol, 3-Methyl-2,4-pentandiol, 2,2-Dimethyl-1,3-propandiol,1-Phenyl-l,2-ethandiol, 3-Methoxy-1,2-propandiol, 3-Phenox-1,2-propandiol, 3-Chlor-l,2-propandiol, 3-Brom-1,2-Propandiol, 3-Buten-1,2-diol, cis- und trans-2-Buten-1,4-diol, Triethanolamin, Triisopropanolamin.

Besonders geeignete Ester sind solche mit der Struktur 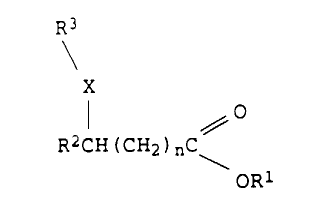 worin
- R¹ =: C₁-C₁₀-Alkyl,
- R² =: C₁-C₁₀-Alkyl, H
- R³ =: H, C₁-C₁₀-Alkyl, gegebenenfalls durch NH₂, OH, C₁₋₄-Alkoxy oder Halogen substituiertes Phenyl,
- X =: O, S, NR⁴,
- R⁴ =: H, C₁-C₁₀-Alkyl, gegebenenfalls durch NH₂, OH, C₁₋₄-Alkoxy oder Halogen substituiertes Phenyl,
- n =: 0,1 oder 2
bedeuten. Unter diesen sind die C₁-₄-Alkylester der C₁-₄-Alkoxyessigsäuren, beispielsweise der Methoxyessigsäure, bevorzugt. Ganz besonders bevorzugt sind die Methyl-, Ethyl-, n-Propyl-, i-Propyl-, n-Butyl-, i-Butyl-, tert. Butylester der Methoxyessigsäure.

Als Hydrolasen können in dem genannten Verfahren eine Vielzahl von Enzymen eingesetzt werden. Bevorzugt werden Proteasen und insbesondere Lipasen verwendet. Als Lipasen sind vor allem mikrobielle Lipasen gut geeignet, die beispielsweise aus Hefen oder Bakterien isolierbar sind. Besonders gut geeignet sind Lipasen aus Pseudomonas, z. B. Amano P oder die Lipase aus Pseudomonas spec. DSM 8246. Weitere besonders gut geeignete Hydrolasen sind die von Novo Nordisk (Enzyme Toolbox) kommerziell erhältlichen Enzyme, insbesondere die Lipasen SP 523, SP 524; SP525, SP 526 und Novozym® 435.

Des weiteren können die Lipasen "Chirazyme L1 bis L8", welche kommerziell erhältlich sind (Boehringer Mannheim), vorteilhaft in dem erfindungsgemäßen Verfahren eingesetzt werden.

Das verwendete Enzym kann in nativer oder in immobilisierter Form eingesetzt werden.

Besonders gut eignet sich das immobilisierte Enzym Novozym® 435.

Als Lösungsmittel sind generell organische Lösungsmittel geeignet. Besonders gut verläuft die Reaktion in Ethern, beispielsweise in MTBE oder THF, oder in Kohlenwasserstoffen wie Hexan, Cyclohexan, Toluol oder halogenierten Kohlenwasserstoffen wie Methylenchlorid.

Die Umsetzung ist aber auch in Abwesenheit eines Lösungsmittels durchführbar.

Die Reaktion verläuft besonders gut, wenn die Lösungsmittel und Einsatzstoffe möglichst wasserfrei vorliegen.

Die Umsetzung des Esters mit dem racemischen Amin bzw. Aminoalkohol unter Enzymkatalyse wird üblicherweise bei Raumtemperatur ausgeführt. Die Reaktionszeiten dafür betragen je nach Substrat 1 bis 48 Stunden. Sekundäre Amine/Aminoalkohole benötigen in der Regel längere Reaktionszeiten als primäre Amine/Aminoalkohole. Die geringere Reaktivität sekundärer Amine kann auch durch eine gegenüber primären Aminen erhöhte Menge an Katalysator ausgeglichen werden.

Pro Mol umzusetzendes Substrat werden 1 bis 6 Mol Ester bevorzugt zugesetzt, d.h. für 1 Mol racemisches Amin benötigt man 0,5 bis3 Mol Ester.

Die zuzusetzende Menge an Enzym hängt von der Art der Hydrolase und der Aktivität der Enzympräparation ab. Die für die Reaktion optimale Enzymmenge kann leicht durch einfache Vorversuche ermittelt werden. In der Regel werden 1000 Units Lipase pro mMol Amin bzw. Aminoalkohol zugesetzt.

Der Reaktionsverlauf läßt sich leicht mit üblichen Methoden beispielsweise mittels Gaschromatographie verfolgen. Im Falle der Racematspaltung beendet man die Reaktion sinnvollerweise bei einem Umsatz von 50 % des racemischen Amins bzw. Aminoalkohols. Dies geschieht in der Regel durch Entfernen des Katalysators aus dem Reaktionsraum, beispielsweise durch Abfiltrieren des Enzyms.

Durch die enantioselektive Umsetzung des racemischen Substrats mit dem Ester entsteht aus dem einen Enantiomer das entsprechend acylierte Produkt (Amid), während das andere Enantiomer unverändert bleibt. Das nun vorliegende Gemisch aus Amin und Amid läßt sich mit üblichen Methoden leicht trennen. Gut geeignet zur Trennung des Gemisches aus Amin und Amid sind beispielsweise Extraktions- oder Destillationsverfahren.

Die anschließende Spaltung des optisch aktiven Amids geschieht nach dem oben beschriebenen Verfahren.

Die folgenden Beispiele dienen der Veranschaulichung der Erfindung.

### Beispiel 1

Enzymatische Acylierung von racemischem Phenylethylamin

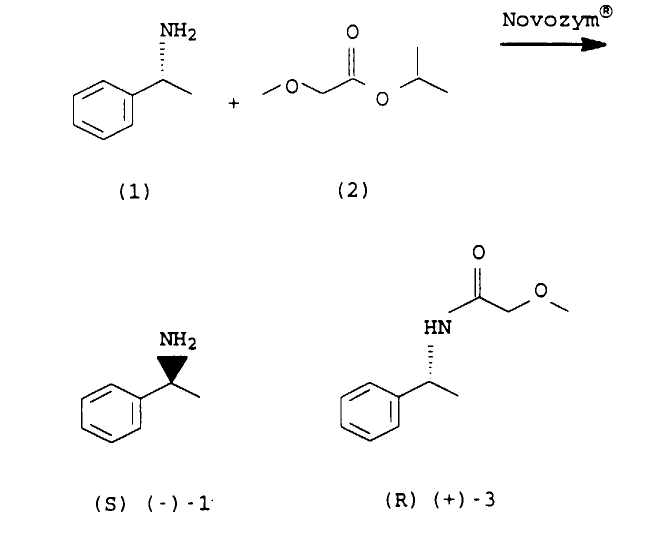

Eine Mischung von 300 g d,l Phenylethylamin (1) und 300 g Methoxyessigsäureisopropylester (2) wird mit Methyl-t-butylether auf ein Gesamtvolumen von 1 l verdünnt. Diese Eduktlösung wird in i einer solchen Geschwindigkeit über einen mit 50 g Novozym® 435 gefüllten Durchflußreaktor gepumpt, daß am Ende des Reaktors ein Umsatz von 50 % erreicht wird.

Die aufgefangene Produktlösung wird im Wasserstrahlpumpenvakuum (Druck 20 mm, Temperatur 35°C) von leicht flüchtigen Bestandteilen befreit. Der Rückstand wird über einen Dünnfilmverdampfer gereinigt, wobei als Destillat eine Mischung des S-Phenylethylamins (-)-1 und nicht-umgesetztes Acylierungsmittel (2) anfällt. Als Rückstand bleibt reines R-Amid(+)-3 mit Fp: 63°C (ee:>99%).

Das S-Amin (-)-1 kann durch fraktionierte Destillation im Wasserstrahlpumpenvakuum vom nicht-umgesetzten Acylierungsmittel befreit werden (Kp =73°C bei 20 mm).

Dabei fällt (-)-1 in einer Enantiomerenreinheit von >99 % an [α]_{D}=-39,5° (pur).

Die Ausbeuten an (-)-1 und (+)-3 liegen über 90 %.

### Beispiel 2

Spaltung des Amids (3)

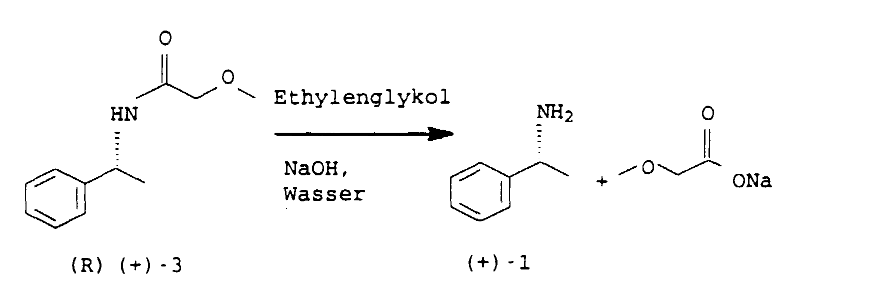

1000 g R-Amid (+)-3 aus Beispiel 1 wurden in 1000g Ethylenglykol suspendiert, auf 170°C erhitzt und so mit 456 g 50%iger wäßriger Natronlauge versetzt. daß die Innentemperatur oberhalb von 150°C bleib. Das freigesetzte Amin (+)-1 geht dabei im Gemisch mit Wasser über (Siedebereich 110-140°C) Nach beendeter NaOH-Zugabe wurden noch 750 ml Wasser in die heiße Mischung eingetropft um restliches Produkt (Amin) überzuschleppen; anschließend wurden im Destillat die Phasen getrennt, die wäßrige Phase noch zweimal mit je 300 ml Toluol extrahiert und die vereinigten organischen Phasen im Vakuum destilliert.

Die wäßrige Phase des Destillats, die noch etwa 2 % Amin enthält, kann erneut zum Schleppen des Amins beispielsweise in einer kontinuierlichen Prozeßfahrweise benützt werden. Die Methoxyessigsäure kann aus dem Rückstand durch Ansäuern zurückgewonnen werden.

Man erhielt 600 g (96 % d.Th.) R-Phenylethylamin (Kp =73°C bei 20 mm; [α]_{D}=30° c=1,0 in Ethanol mit ee> 99%.

## Patentansprüche

1. Verfahren zur Spaltung von optisch aktiven Amiden zu Carbonsäuren und optisch aktiven Aminen unter Erhalt des Chiralitätszentrums, dadurch gekennzeichnet, daß man eine Hydrolyse der Amide in Gegenwart eines Polyols oder eines Aminoalkohols und eines Alkali- oder Erdalkalihydroxids durchführt.

2. Verfahren nach Anspruch 1, dadurch gekennzeichnet, daß das Polyol oder der Aminoalkohol in einer Menge von 10 bis 90 Gew.-%, bezogen auf das gesamte Lösungsmittel, bei der Hydrolyse verwendet wird.

3. Verfahren nach Anspruch 1, dadurch gekennzeichnet, daß als Polyol Ethylenglykol verwendet wird.

4. Verfahren nach Anspruch 1, dadurch gekennzeichnet, daß als Alkalihydroxid NaOH oder KOH eingesetzt wird.

5. Verfahren nach Anspruch 1, dadurch gekennzeichnet, daß die Hydrolyse bei einer Temperatur oberhalb von 100°C durchgeführt wird.

6. Verfahren nach einem der oben genannten Ansprüche als Bestandteil eines Verfahrens zur Racematspaltung von primären und sekundären Aminen, das folgende Schritte umfaßt:
1. Umsetzung der racemischen Amine mit einem Ester, dessen Säurekomponente ein Fluor-, Stickstoff-, Sauerstoff-, Phosphor- oder Schwefelatom, gebunden an ein Kohlenstoffatom in alpha, beta oder gamma Position zum Carbonylkohlenstoffatom, trägt, unter spezifischer Katalyse mit einer Hydrolase,
2. Trennung des einen, enantioselektiv acylierten Amins vom nicht umgesetzten anderen Enantiomer des Amins,
3. anschließende Hydrolyse des acylierten Amins nach einem der oben beanspruchten Verfahren.

## Claims

1. A process for cleaving optically active amides to carboxylic acids and optically active amines with retention of the center of chirality, wherein the amides are hydrolyzed in the presence of a polyol or of an amino alcohol and of an alkali metal or alkaline earth metal hydroxide.

2. A process as claimed in claim 1, wherein the polyol or amino alcohol is used in the hydrolysis in an amount of from 10 to 90 % by weight, based on the entire solvent.

3. A process as claimed in claim 1, wherein ethylene glycol is used as polyol.

4. A process as claimed in claim 1, wherein NaOH or KOH is employed as alkali metal hydroxide.

5. A process as claimed in claim 1, wherein the hydrolysis is carried out at a temperature above 100°C.

6. A process as claimed in any of the abovementioned claims as part of a process for resolving the racemates of primary and secondary amines, which comprises the following steps:
1. reaction of the racemic amines with an ester whose acid component has a fluorine, nitrogen, oxygen, phosphorus or sulfur atom bonded to a carbon atom in the position alpha, beta or gamma to the carbonyl carbon, with specific catalysis by a hydrolase,
2. separation of the enantioselectively acylated amine from the other, unreacted, enantiomer of the amine,
3. subsequent hydrolysis of the acylated amine by one of the processes claimed above.

## Revendications

1. Procédé pour décomposer des amides possédant l'activité optique en acides carboxyliques et amines possédant l'activité optique avec conservation du centre de chiralité, caractérisé par le fait que l'on procède à une hydrolyse des amides en présence d'un polyol ou d'un aminoalcool et d'un hydroxyde alcalin ou alcalino-terreux.

2. Procédé selon revendication 1, caractérisé par le fait que le polyol ou l'aminoalcool est utilisé à l'hydrolyse en quantité de 10 à 90 % du poids du solvant total.

3. Procédé selon revendication 1, caractérisé par le fait que le polyol utilisé est l'éthylèneglycol.

4. Procédé selon revendication 1, caractérisé par le fait que l'hydroxyde alcalin utilisé est NaOH ou KOH.

5. Procédé selon revendication 1, caractérisé par le fait que l'hydrolyse est réalisée à une température supérieure à 100° C.

6. Procédé selon une des revendications qui précèdent en tant que partie d'un procédé pour résoudre les racémates d'amines primaires et secondaires comportant les stades opératoires suivants :
1. réaction des amines racémiques avec un ester dont le composant acide porte un atome de fluor, d'azote, d'oxygène, de phosphore ou de soufre relié à un atome de carbone en position alpha, bêta ou gamma de l'atome de carbone de carbonyle, avec catalyse spécifique par une hydrolase,
2. séparation de l'une des amines, ayant subi une acylation énantiosélective, de l'autre énantiomère de cette amine, qui n'a subi aucune conversion,
3. hydrolyse subséquente de l'amine acylée selon l'un des procédés revendiqués ci-dessus.
